(19) Europäisches Patentamt
European Patent Office
Office européen des brevets

(11) Publication number: **0 272 446 B1**

(12) **EUROPEAN PATENT SPECIFICATION**

(45) Date of publication of patent specification: **11.11.92**

(51) Int. Cl.[5]: **C07F 9/564**, A61K 31/675

(21) Application number: **87116868.8**

(22) Date of filing: **14.05.84**

(60) Publication number of the earlier application in accordance with Art.76 EPC: **0 145 739**

(54) **Novel antiviral agents.**

(30) Priority: **24.05.83 US 497720**

(43) Date of publication of application: **29.06.88 Bulletin 88/26**

(45) Publication of the grant of the patent: **11.11.92 Bulletin 92/46**

(84) Designated Contracting States: **FR**

(56) References cited:
**EP-A- 0 015 584**
**EP-A- 0 049 072**
**EP-A- 0 074 306**
**DE-A- 2 009 834**
**DE-A- 3 045 375**
**FR-A- 2 381 781**
**GB-A- 1 243 213**
**US-A- 3 446 793**
**US-A- 3 560 478**

**Alfred Burger ; Medical Chemistry, 3rd edition, part 1, pages 73-79 (1979)**

**Fuertes ; 3. Med. Chem. 17(6), p. 642 (1974)**

**Journal of Medicinal Chemistry, vol. 22, no. 1, January 1979, J.A. Montgomery et al.: "Phosphonate analogue of 2'-Deoxy-5-fluorouridylic acid", pages 109-111, see pages 109-110**

(73) Proprietor: **SRI INTERNATIONAL**
**333 Ravenswood Avenue**
**Menlo Park, California 94025(US)**

(72) Inventor: **Reist, Elmer Joseph**
**581 Berkeley Avenue**
**Menlo Park California 94025(US)**
Inventor: **Sturm, Priscilla Anna**
**1549 Montalvo Drive**
**Mountain View California 94040(US)**

(74) Representative: **Santarelli, Marc**
**Cabinet Rinuy et Santarelli 14, avenue de la Grande Armée**
**F-75017 Paris(FR)**

**EP 0 272 446 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Journal of Heterocyclic Chemistry, vol. XI, February, April, June 1974, J.A. Montgomery et al.: "The use of the wittig reaction in the modification of purine nucleosides (1)", pages 211-218, see page 213

Journal of the American Chemical Society, vol. 92, no. 18, 9 September 1970, G.H. Jones: "Communications to the editor. Synthesis of Isoteric Phosphonate analogs of some biologically important phosphodiesters", pages 5510-5511, see pages 5510-5511

Journal of the American Chemical Society, vol. 90, no. 19, 11 September 1968, G.H. Jones et al.: "The Synthesis of 6'-Deoxyhomonucleoside-6'-phosphonic Acids", pages 5337-5338, see pages 5337-5338

Liebigs Annalen der Chemie, vol. 1, 1984, Verlag Chemie (Weinheim, DE), J. Hollmann et al.: "Darstellung und Konformationszuordnung einiger 5'-homologer Adenosinderivate", pages 98-107, see page 99

Chemical Review, no. 3, June 1977, R. Engel: "Phosphonates as analogues of natural phosphates", pages 349-367, see page 351

Biochemistry, vol. 12, no. 9, 24. April 1973; A. Hampton et al.: "Synthesis of homoadenosine-6'-phosphonic acid and studies of its substrate and inhibitor properties with adenosine monophosphate utilizing enzymes", pages 1730-1736, see page 1732

## Description

### Field of the Invention

This invention concerns nucleotide analogues and their synthesis and use. More particularly, it concerns phosphonic acid analogues of natural and synthetic nucleoside phosphates and their preparation and use as antiviral agents.

The present patent application is a division of the European patent application 84 902178.7 filed on 14 May 1984, published under No. 0145,739 and now granted.

### Background of the Invention

There is a recognized need for antiviral agents. Herpes virus hominis alone infects between 50 and 150 million Americans at this time. A number of the antiviral agents that are currently viewed as most effective against herpes are nucleoside analogues. These materials include iododeoxyuridine, 2-hydroxyethoxymethylguanine, 2'-fluoro-5-iodo-1-arabinofuranosyl cytosine and 5-E-bromovinyldeoxyuridine. It is believed that these materials act through their conversion by viral thymidine kinase (but not by host TK) to the nucleotide which is then converted to the triphosphate and incorporated into viral DNA. The incorporation of these analogues into the viral DNA prevents its replication and thus is lethal to the virus. Two shortcomings of this antiviral mechanism have been recognized, however. First, thymidine kinase negative herpes mutants ($TK^-$) have been identified which are inherently inactive toward phosphorylating these analogues and thus permitting their incorporation in viral DNA. In addition, $TK^+$ mutants that are resistant to 2-hydroxyethoxymethylguanine have been reported in mice by H. Field, et al, in J Infect Dis, 143 281 (1981). $TK^+$ mutants resistant to iododeoxyuridine have been reported in humans by A. Hirano, et al, in Acta Virol 23 226 (1979). It may be that these newly-discovered resistant viral strains do not undergo the monophosphorylation or triphosphate formation needed to permit incorporation in the DNA.

References to these antiviral agents of the art and their use include Am J Med, 73 No 1A, July 20, 1982 "Proceedings of a Symposium on Acyclovir"; Biochem Biophys Acta, 32 295-6 (1959); Antimicrob Agents Chemother, 578-584 (1965); Science, 145 585-6 (1964); Science, 255 468-80 (1975); J Med Chem, 19 495-8 (1976); Proc Natl Acad Sci, 76 4415-18 (1979); and J Med Chem 22 21-24 (1979).

The present invention provides antiviral materials which can be lethally incorporated into DNA without the dependence upon enzyme-moderated phosphorylation. The materials of the invention are phosphonate analogues of the mono-, di- and triphosphates of the deoxynucleotide analogs. An article by Robert Engel appearing at Chem Reviews, 11, #3 pp 349-367 (1977) discusses phosphonate analoges of nucleotides and the like. Other representative references in this area, some of which are cited in the Chem Reviews article, are German 0.L.S 2,350,608 (1974) of Syntex (Jones and Moffatt inventors); German O.L.S. 2,009,834 1970 also of Syntex with Jones and Moffatt as inventors; British Patent 1,243,214 of Syntex, and US Patent 3,560,478 of Myers.

### Statement of the Invention

A group of new materials have now been found. These materials in a broad sense are phosphonate analogues of mono-, di-, and triphosphates of antiviral nucleoside analogues. These analogues differ from nonlethal natural nucleosides by variations in their sugar ribose moiety and/or by variations in their nucleoside base moieties. Such materials are represented structurally as

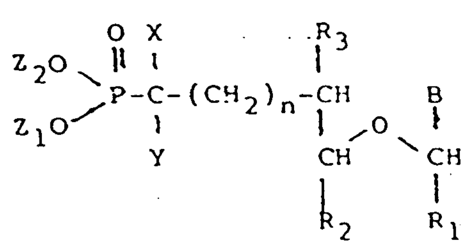

wherein $Z_1$ and $Z_2$ are the same or different and selected from the group made up of hydrogen, the one to six carbon alkyls, phenyl and benzyl, X is H, OH, or together with Y = O, Y is H or together with X = O, n

is an integer, 0, 2 or 4, $R_1$ and $R_2$ together complete a $\beta$-pentofuranose sugar or $R_1$ is H and $R_2$ is H or $-CH_2OH$, $R_3$ is H or OH and B is a pyrimidine base selected from 5-iodouracil, 5-trifluorothymine, 5-iodocytosine, E-5-(2-bromo-vinyl) uracil, 5-propyluracil and 5-ethyluracil.

In other aspects this invention relates to the preparation of these materials, their formulation into antiviral pharmaceutical compositions and the use of these formulations to treat viral infections, in particular herpes infections.

## Detailed Description of the Invention

### The compounds

The compounds of this invention are phosphonates which have the structure set forth above in Statement of the Invention.

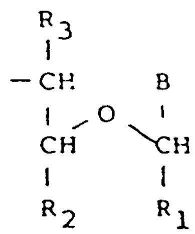

The unit defines an antiviral nucleoside.

As previously noted, in this structure $R_1$ and $R_2$ can together complete a $\beta$-pentofuranose sugar. In this configuration they preferably complete a substituted or unsubstituted $\beta$-ribofuranose or $\beta$-arabinofuranose such as ribose, 2-deoxyribose, 2,3-dideoxyribose, 3-deoxyribose, 2-fluoro-2-deoxyribose or arabinose or the like.

In this structure, $Z_1$ and $Z_2$ preferably are each selected from hydrogens and one to four carbon alkyls. More preferably $Z_1$ and $Z_2$ are each hydrogens. Further in this structure, the integer n is significant in defining whether the compound is in size equivalent to a nucleoside monophosphate, a diphosphate or a triphosphate.

Preferred bases include 5-iodouracil, 5-trifluorothymine, 5-iodocytosine, E-5-2-bromovinyluracil, 5-propyluracil, and 5-ethyluracil.

Preferred nucleoside analogues (e.g.

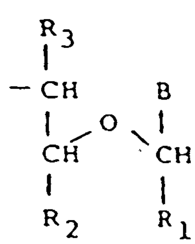

include 5-iodo-2'-deoxyuridine, 5-trifluorothymidine, E-5-(2-bromovinyl)-2'-deoxyuridine, 1-(2'-deoxy-2'-fluoro-B-D-arabinofuranosyl)-5-iodocytosine, 5-ethyl-2'-deoxyuridine, 5-propyl-2'-deoxyuridine.

These nucleoside analogues yield phosphonates (and di- and triphosphate-phosphonate analogues) having the following general structures

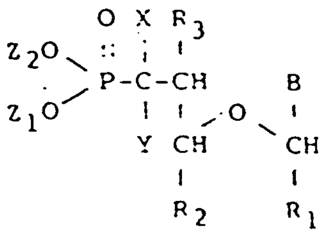

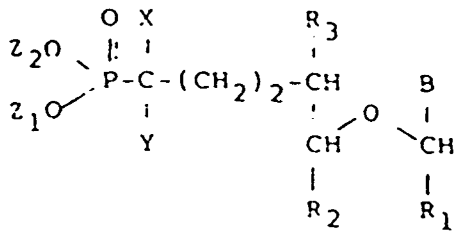

and

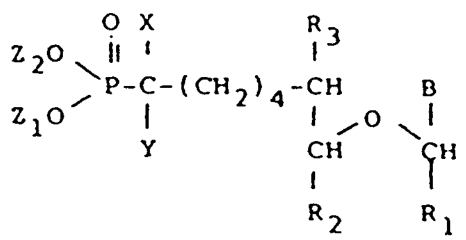

wherein X, Y, $Z_1$, $Z_2$, B, $R_1$, $R_2$ and $R_3$ are as described above. In preferred embodiments, X is hydrogen or hydroxyl and Y is hydrogen. Thus, representative compounds can have the structures shown in Table I.

Table I

Representative Structures

| Structure | Structure Number |
|---|---|
| 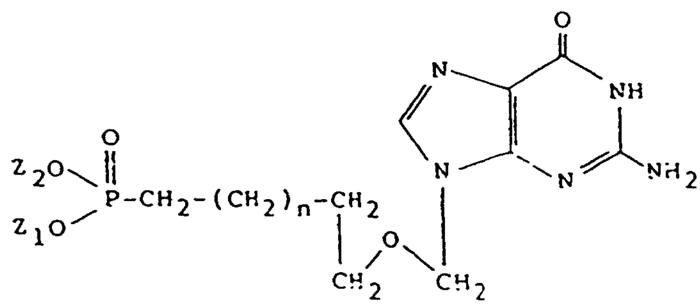 | 1 |
| 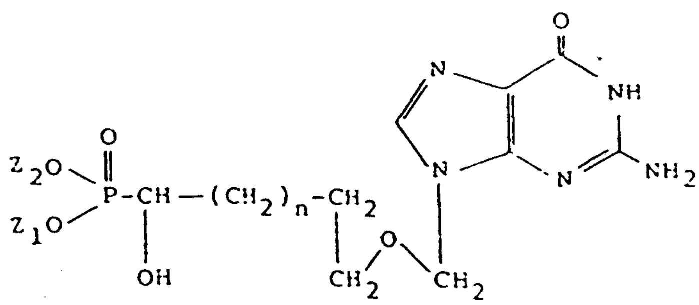 | 2 |
| 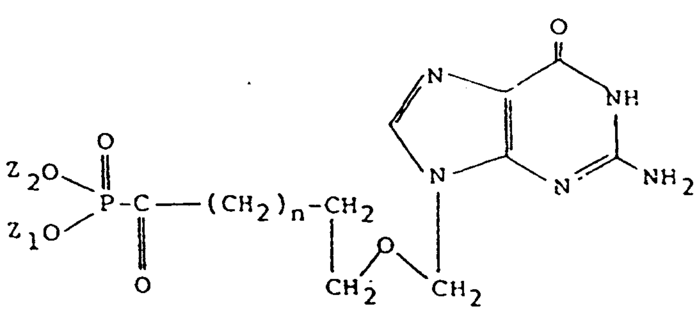 | 3 |

* (to be applied to the claimed subject-matter "mutatis mutandis")

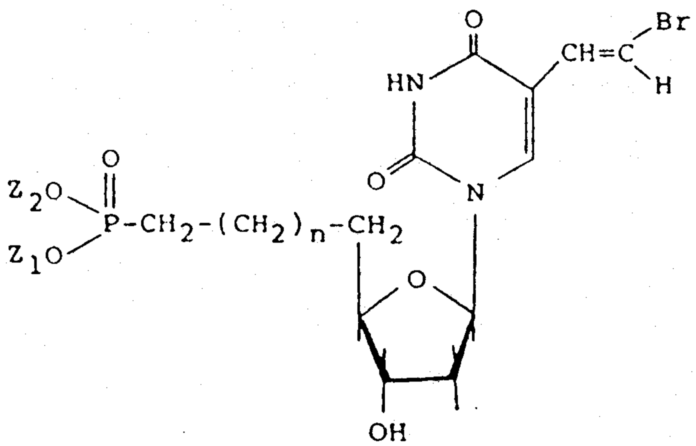

4

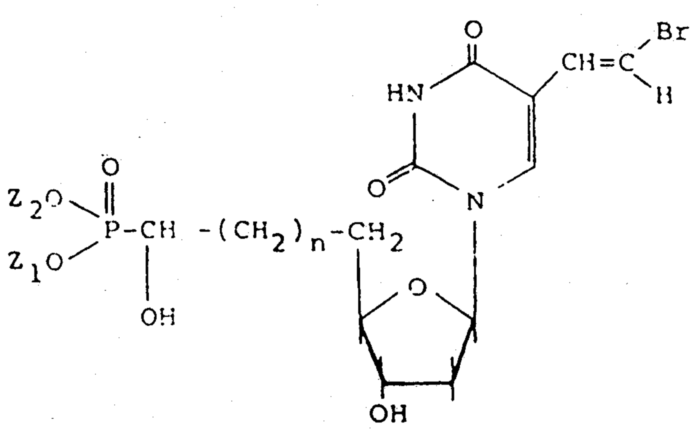

5

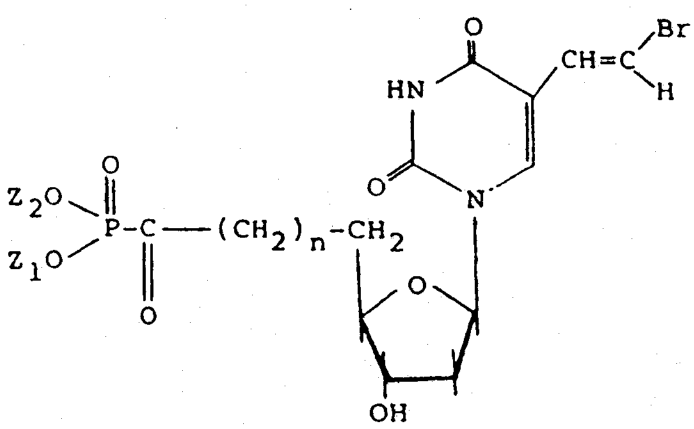

6

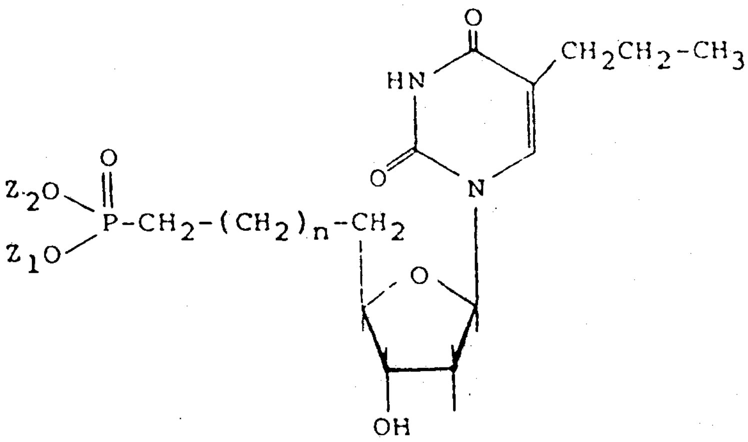

7

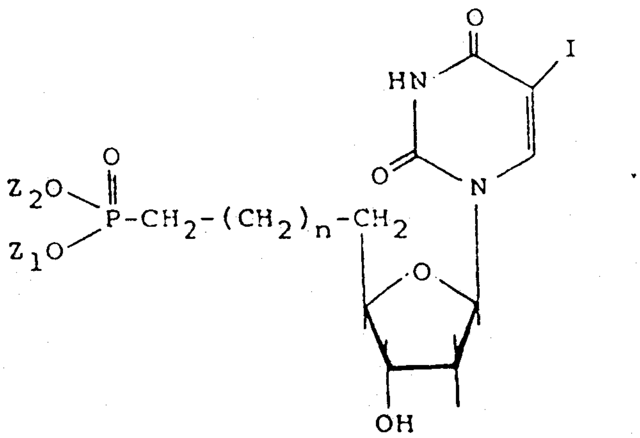

8

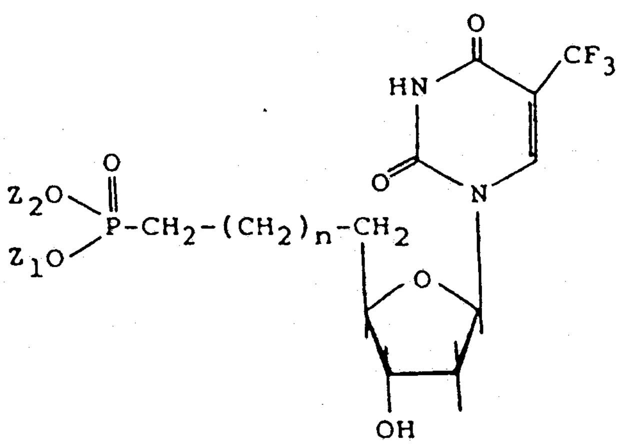

9

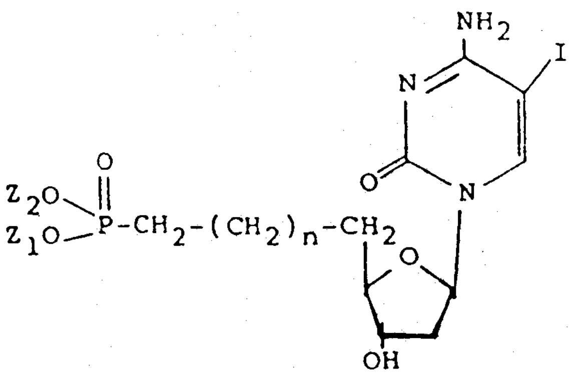

10

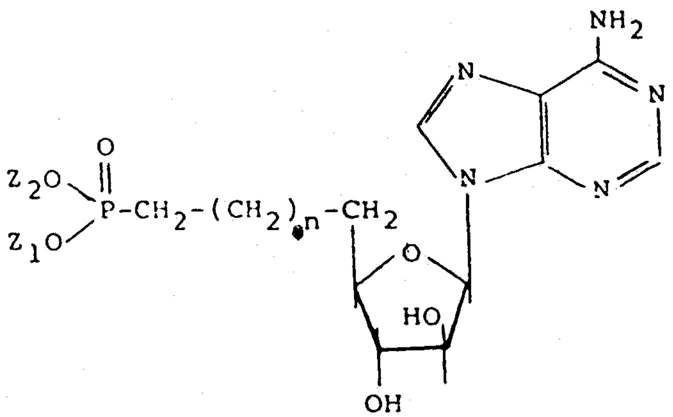

11

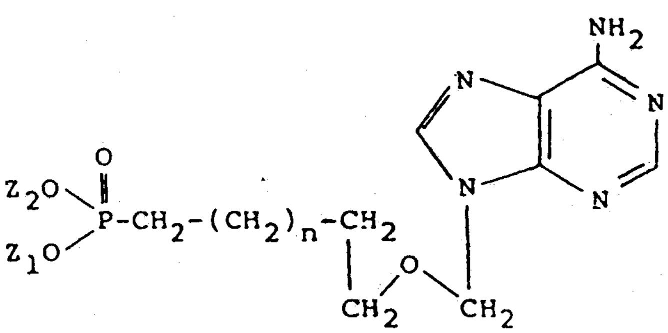

12

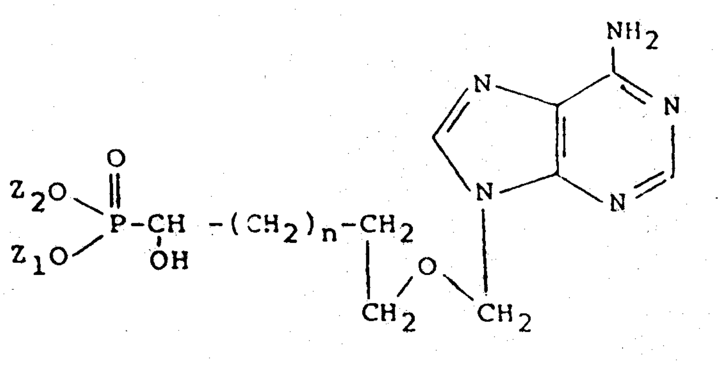

13

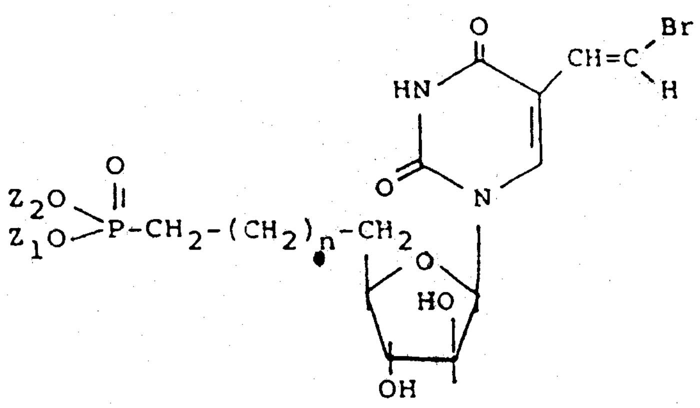

14

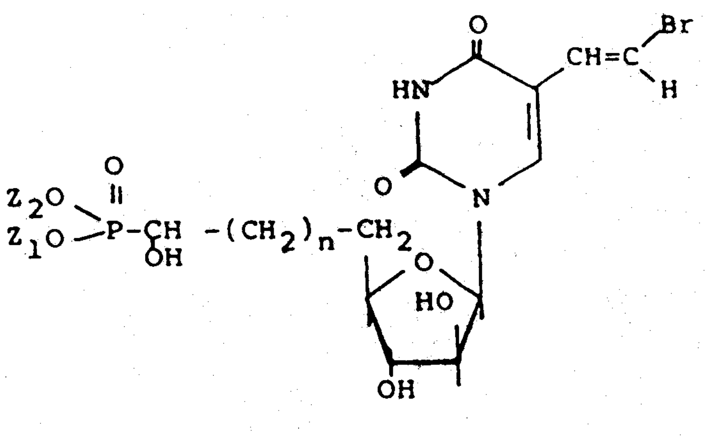

15

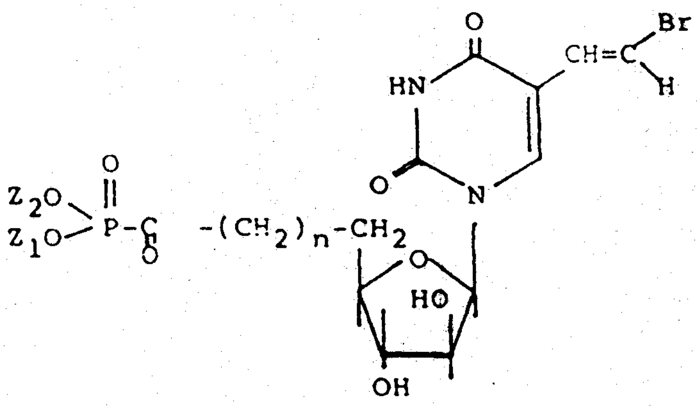

16

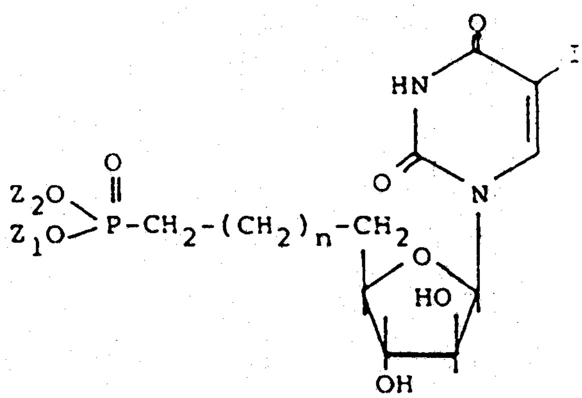

17

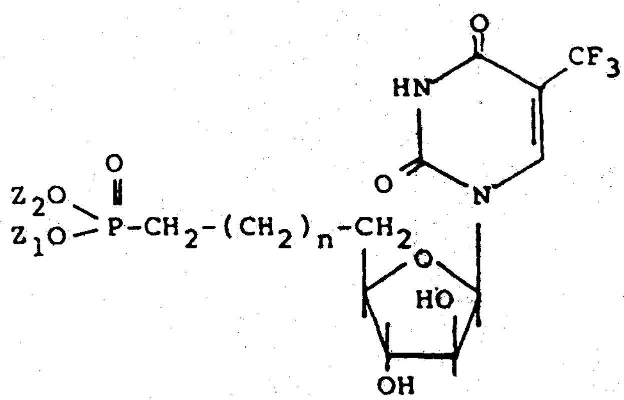

18

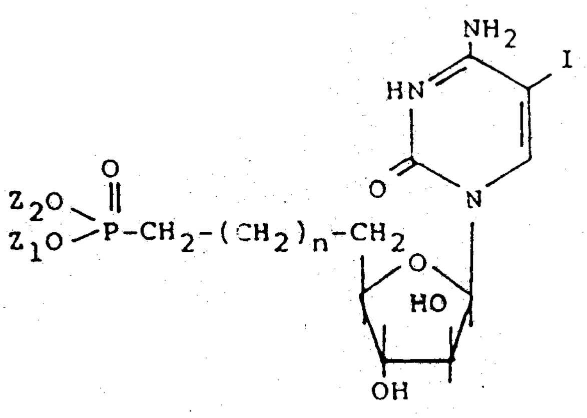

19

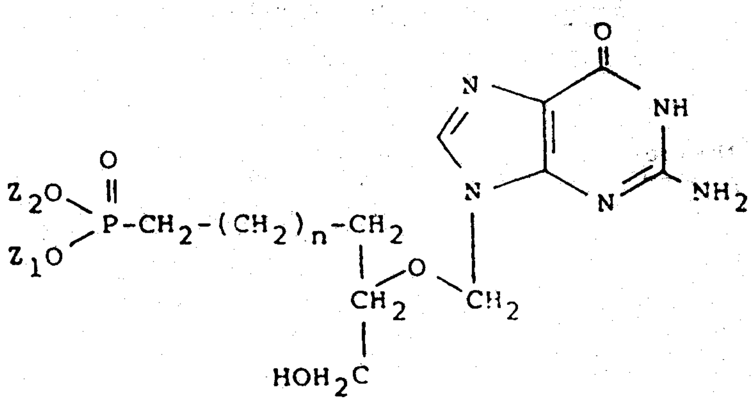

20

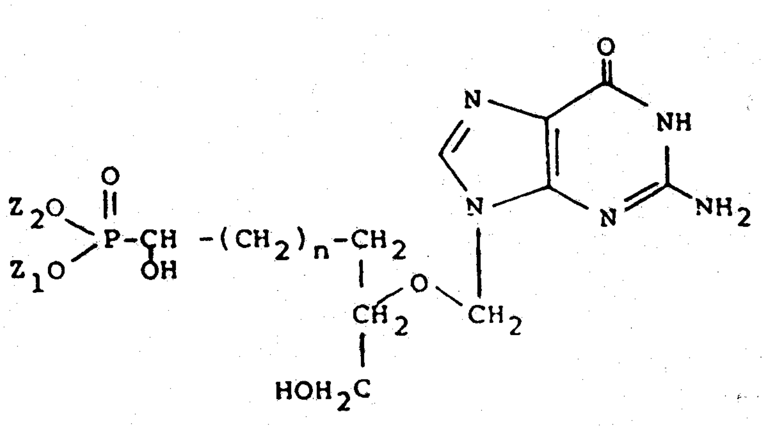

21

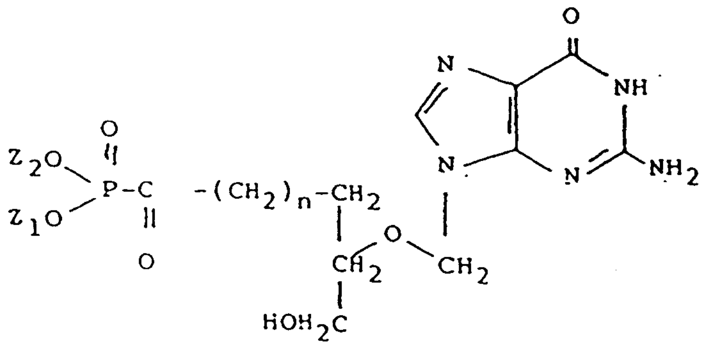

22

Table II

| Representative Compounds* Monophosphate Analogues | | | | |
|---|---|---|---|---|
| Compound Number | Structure Number | $Z_1$ | $Z_2$ | n |
| 1a | 1 | H | H | O |
| 1b | 1 | $CH_3$ | $CH_3$ | O |
| 1c | 1 | H | $CH_3$ | O |
| 1d | 1 | H | $C_2H_5$ | O |
| 2a | 2 | H | H | O |
| 2b | 2 | $CH_3$ | $CH_3$ | O |
| 2c | 2 | H | $CH_3$ | O |
| 2d | 2 | H | $C_2H_5$ | O |
| 3a | 3 | H | H | O |
| 3b | 3 | $CH_3$ | $CH_3$ | O |
| 3c | 3 | H | $CH_3$ | O |
| 3d | 3 | H | $C_2H_5$ | O |
| 22a | 22 | H | H | O |
| 22b | 22 | $CH_3$ | $CH_3$ | O |
| 22c | 22 | H | $CH_3$ | O |
| 22d | 22 | H | $C_2H_5$ | O |

* (to be applied to the claimed subject-matter "mutatis mutandis")

| Diphosphate Analogs | | | | |
|---|---|---|---|---|
| Compound number | Structure Number | $Z_1$ | $Z_2$ | n |
| 1e | 1 | H | H | 2 |
| 1f | 1 | $CH_3$ | $CH_3$ | 2 |
| 1g | 1 | H | $CH_3$ | 2 |
| 1h | 1 | H | $C_2H_5$ | 2 |
| 2e | 2 | H | H | 2 |
| 2f | 2 | $CH_3$ | $CH_3$ | 2 |
| 2g | 2 | H | $CH_3$ | 2 |
| 2h | 2 | H | $C_2H_5$ | 2 |
| 22a | 22 | H | H | 2 |
| 22b | 22 | $CH_3$ | $CH_3$ | 2 |
| 22c | 22 | H | $CH_3$ | 2 |
| 22d | 22 | H | $C_2H_5$ | 2 |

| Triphosphate Analogues | | | | |
|---|---|---|---|---|
| Compound Number | Structure Number | $Z_1$ | $Z_2$ | n |
| 1 | 1 | H | H | 4 |
| 1 | 1 | $CH_3$ | $CH_3$ | 4 |
| 1 | 1 | H | $CH_3$ | 4 |
| 1 | 1 | H | $CH_3$ | 4 |
| 22 | 22 | H | H | 4 |
| 22 | 22 | $CH_3$ | $CH_3$ | 4 |
| 22 | 22 | H | $CH_3$ | 4 |
| 22 | 22 | H | $C_2H_5$ | 4 |

These are merely representative compounds as it will be apparent to those skilled in the art that other combinations of substituents and bases could be employed as well.

Preparation

The compounds of this invention can be prepared by the following general procedures: The non β-pentofuranose materials such as materials having structures 1, 2, 12, 13, 20, 21, and 22 in Table I can be made with the representative reaction sequence I.

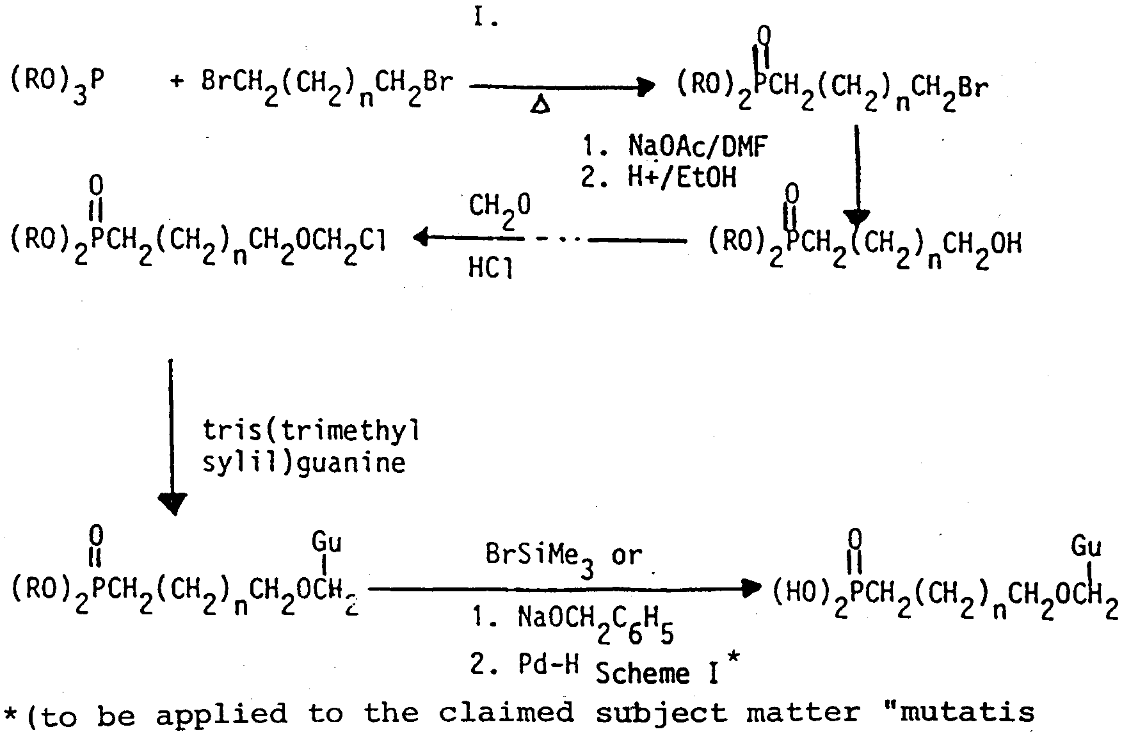


*(to be applied to the claimed subject matter "mutatis mutandis")

In this sequence, a trialkyl ($C_2$, $C_4$ or $C_6$) phosphite reacts with a dibromoalkane in an Arbuzov reaction to give the bromoalkyl phosphonate (See J Am Chem Soc, 87 (2), 253 (1965)). (All cited references are incorporated herein).

Displacement of the bromide using sodium acetate in DMF followed by hydrolysis of the acetate ester gives diethyl 3-hydroxypropylphosphonate (i.e. in sequence I, R = $C_2H_5$, n = 1). This material is chloromethylated to the chloromethylester and then reacted with a suitably protected base such as tristrimethylsilylguanine by the method of Kelley, et al. J Med Chem, 24 1528 (1981). This yields the phosphono product, e.g. 9-(3-phosphonopropyloxymethyl)guanine, directly. Phosphonate esters are smoothly cleaved by bromotrimethylsilane to the phosphonic ester by the method of McKenna, et al, Tet Letts, 155

(1977).

The syntheses of a representative deoxyriboside is illustrated in Scheme II.

Oxidation of 2′, 3′-0-isopropylidine-5-propyluridine (1) by the Moffatt procedure, Pfitzner and Moffatt J Am Chem Soc, 85 3027 (1963) will yield the 5′-aldehyde. The reaction of (2) by the Wittig reagent prepared as shown in Scheme (2) gives the chain-extended, unsaturated "nucleotide" (4). Hydrogenation and deacetonation of the nucleotide (4) will give the partially unblocked nucleotide (5).

The conversion of the riboside (5) to the deoxyriboside (7) is accomplished by a strategy desscribed recently by Lessor and Leonard, J Org Chem, 46 4300 (1981), which was based on the selective partial deacylation of fully acylated nucleosides outlined by Ishido, et al, J Chem Soc, Perk I, 563 (1980). Thus benzoylation of (5) to the 2′, 3′-di-O-benzoate followed by treatment with hydroxylaminium acetate in dry pyridine will give the 3′-benzoate (6). Thiobenzoylation of (6) followed by treatment with tributyl tin and debenzoylation using sodium benzyloxide, converts the 2′-hydroxyl to H, deacylates the 3′-O-benzoate, and substitutes the phenyl phosphate ester with benzyl phosphate, Jones and Moffatt J Am Chem Soc, 90 5337 (1968). Hydrogenolysis removes the benzyl ester and gives the desired phosphonic acid (7).

Alternatively, compounds in the 2-deoxyribose series are prepared from the 2′-deoxynucleoside by chemistry outlined in Scheme III. Thus, selective tritylation follwoed by mesylation of 5-propyl-2′-deoxyuridine (1) gives (8), which is converted to the 2,3′-cyclonucleoside (9) using sodium benzoate in DMF (Yung and Fox, J Am Chem Soc, 83 3060 (1961)). Oxidation to the aldehyde (10) followed by a Wittig condensation gives the unsaturated phosphonate (11). Hydrogenation and ring-opening (Yung and Fox) gives the phosphonate ester (7), which can be deblocked, (U.S. Patent 3,524,846 and J Am Chem Soc, 90 5337 (1968)) to the free phosphonic acid. Hydroboration of (11) followed by treatment with sodium benzoate in dimethyl formamide (DMF) gives a mixture of 5′-hydroxy and 6′-hydroxy isomers.

Oxygen functionality $\alpha$ to the phosphonate can be introduced by the chemistry outlined in Scheme IV. The appropriate propargyl phosphonic acid (13) can be prepared from tetrahydropyranyl-propargyl alcohol (12) by the procedures outlined by Chattha and Aquiar, J Org Chem. 36 2719-20 (1971). Selective hydrogenation to the vinyl phosphonate followed by the sequence outlined in Scheme IV results in phosphonate analogues with one or two hydroxyls in the phosphonate chain.

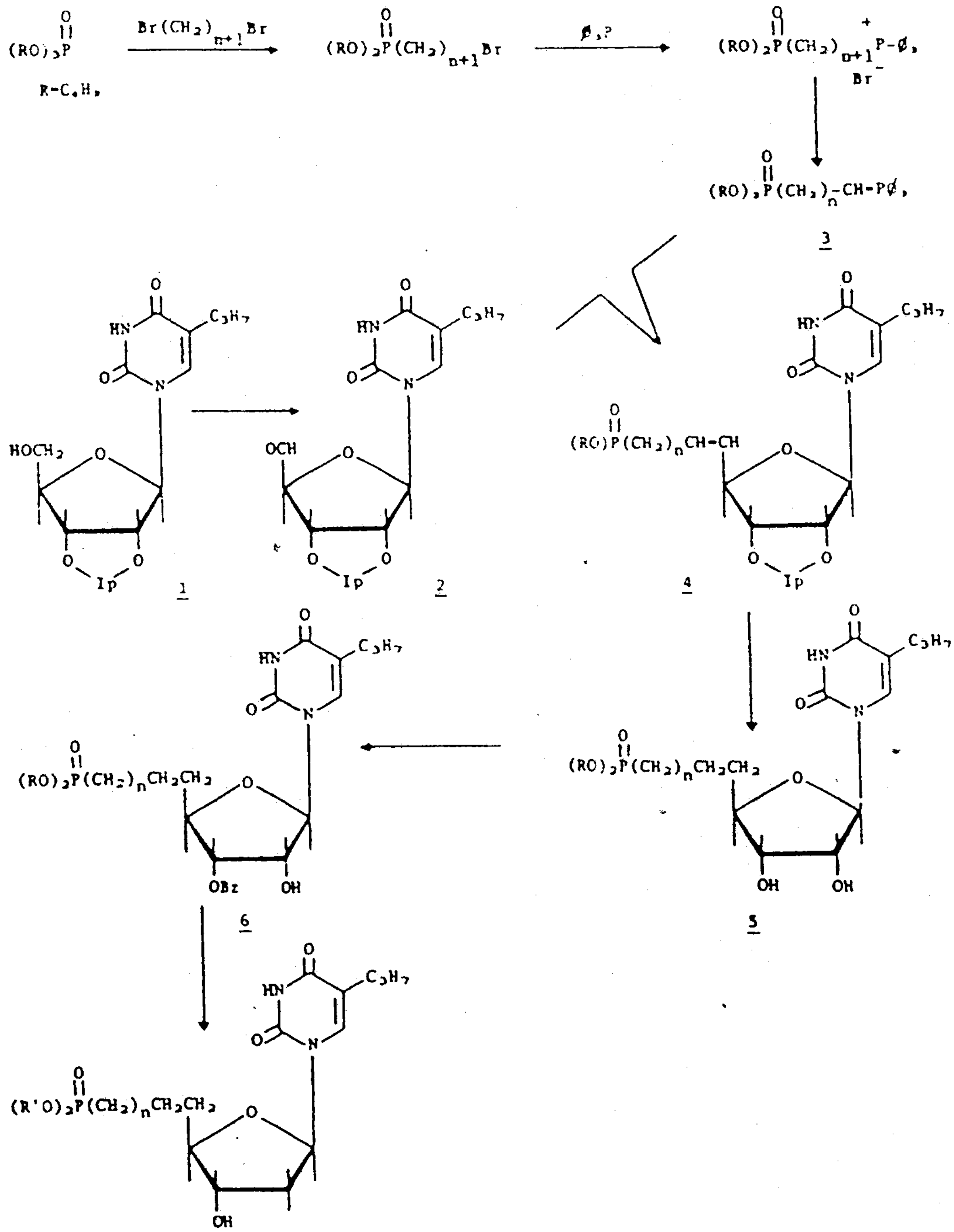


7 n=O, 2, 4
R'=$C_6H_5$, H

Scheme II

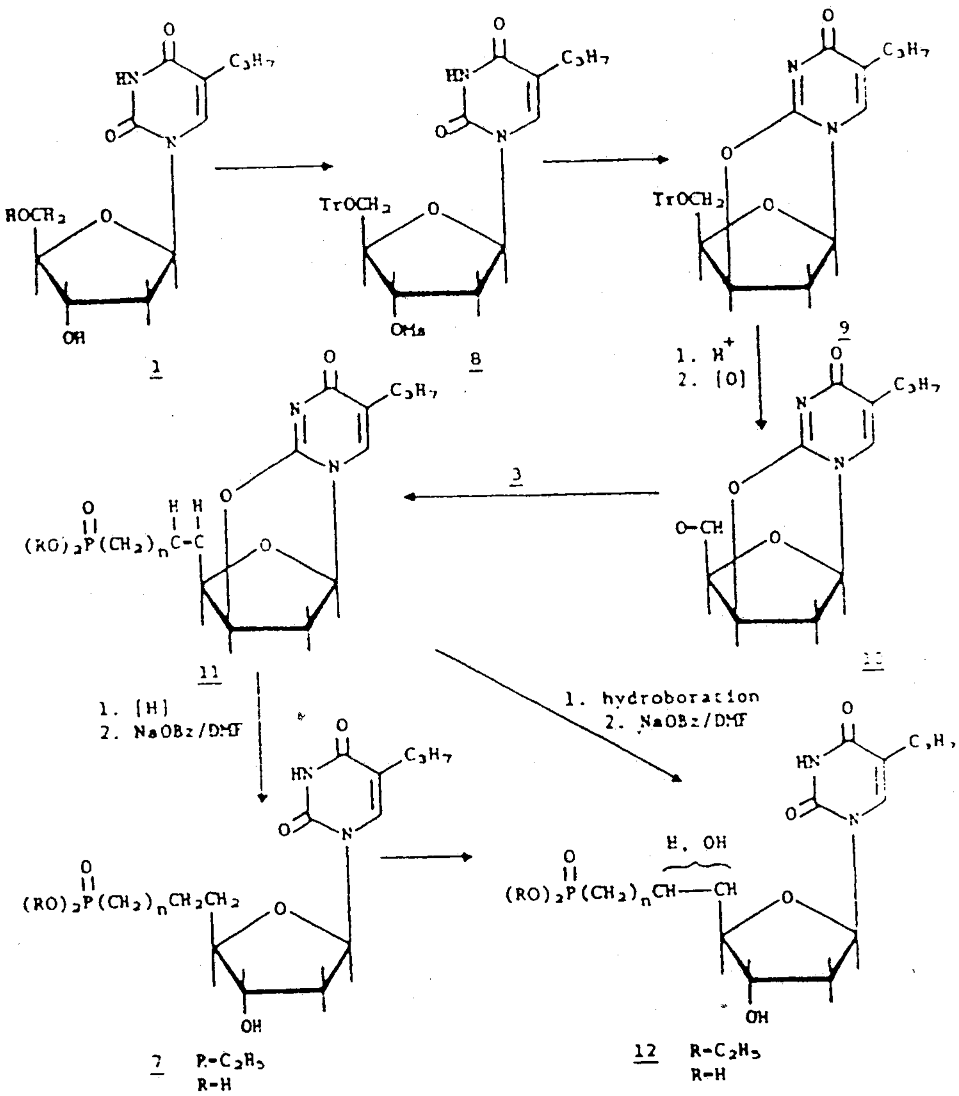
1
8
9
1. H+
2. [O]
3
11
10
1. [H]
2. NaOBz/DMF
1. hydroboration
2. NaOBz/DMF
7 R=C2H5
R=H
12 R=C2H5
R=H

Scheme III

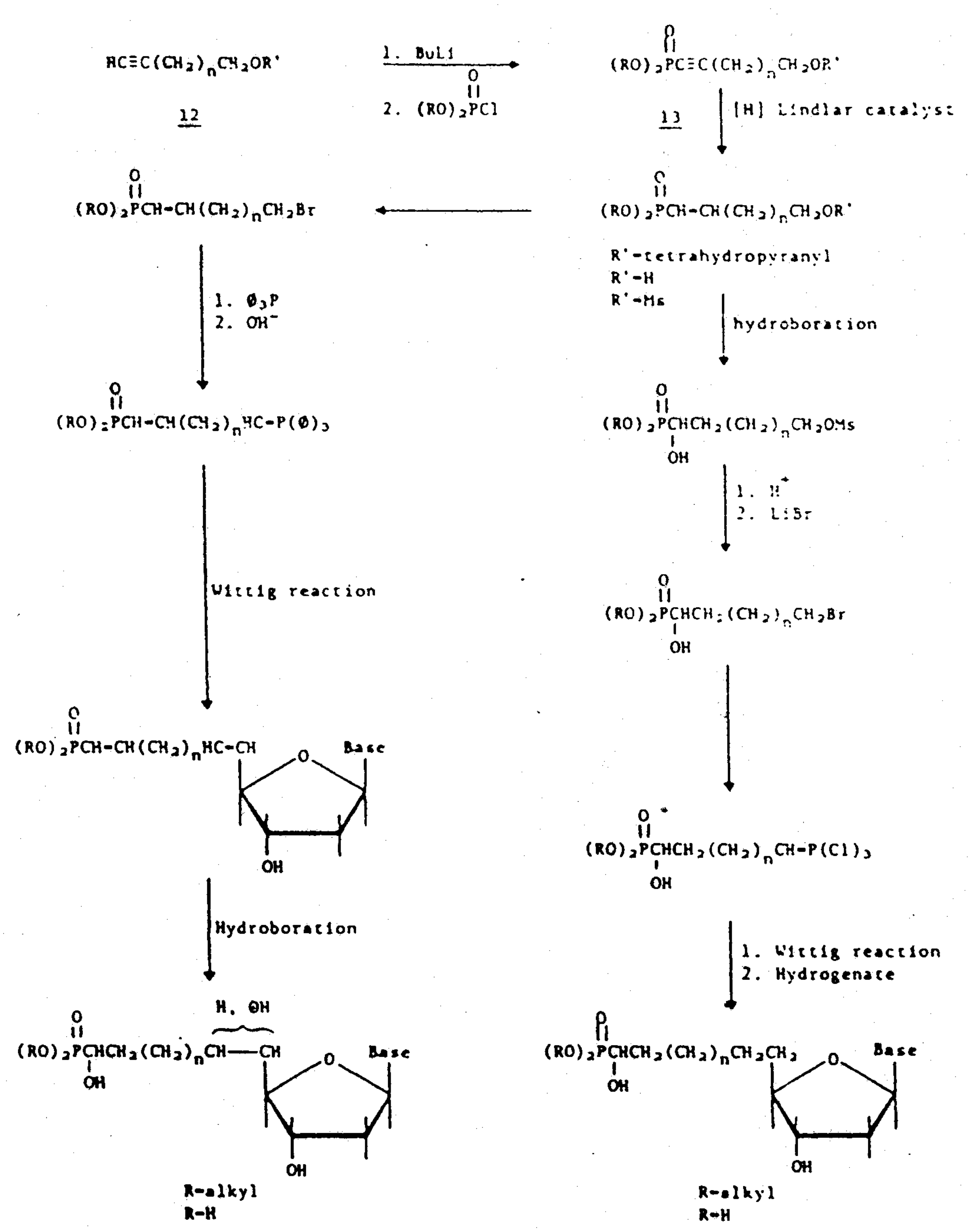


Scheme IV

Carbonyl analogs can be prepared by the chemistry outlined in Scheme V. This method involves Arbuzov reaction of triethylphosphite with $\beta$-acetoxypropionyl chloride (14) by the method of Yamashita, et al, Bull Chem Soc Japan, 53(6) 1625 (1980).

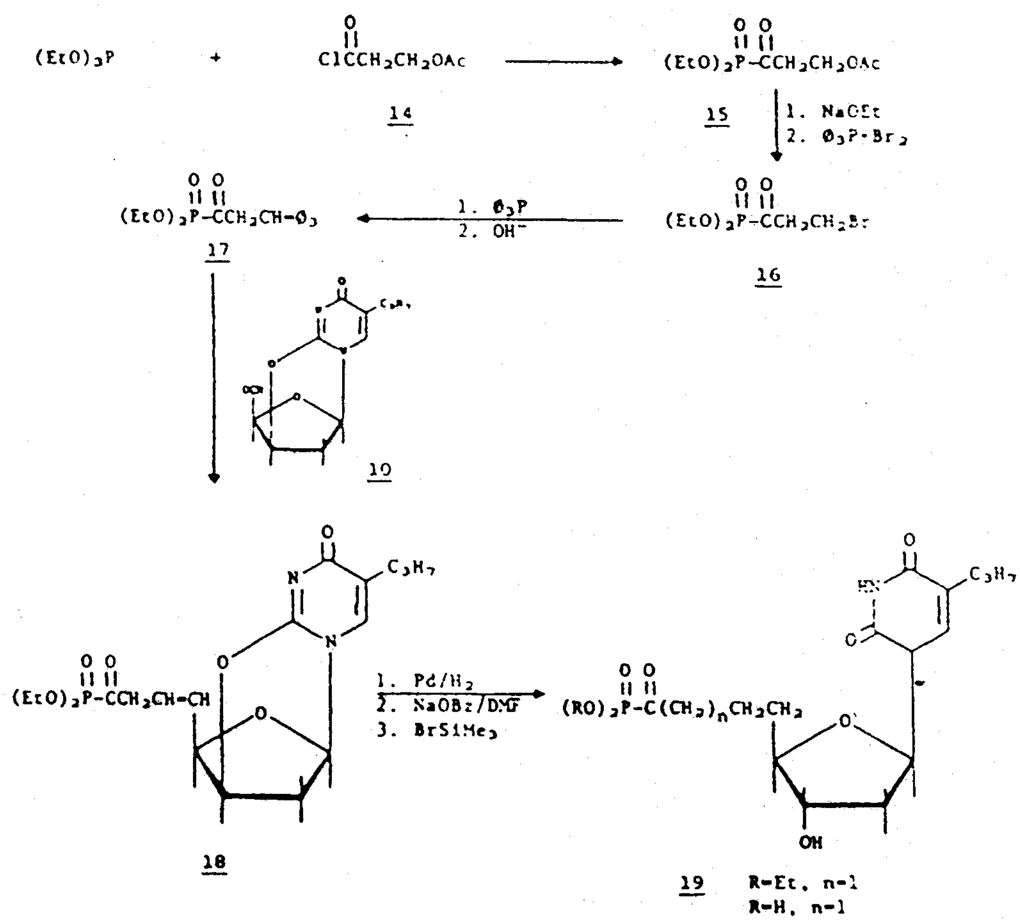


Scheme V

This gives the α-carbonyl phosphonate ester (15). Conversion of 15 to the bromoethyl derivative 16 followed by reaction with triphenylphosphine will give the Wittig reagent (17). Condensation of 17 with the appropriate aldehyde (e.g. 10) gives the olefin 18, which, after hydrogenation and deblocking, results in the desired product 19, a nucleoside diphosphonate analog. The nucleoside triphosphate analog 19 (wherein n = 3) can be prepared starting from 5-acetoxyvalerylchloride.

The nucleoside monophosphonate analog (20) can be prepared by the chemistry outlined in Scheme VI.

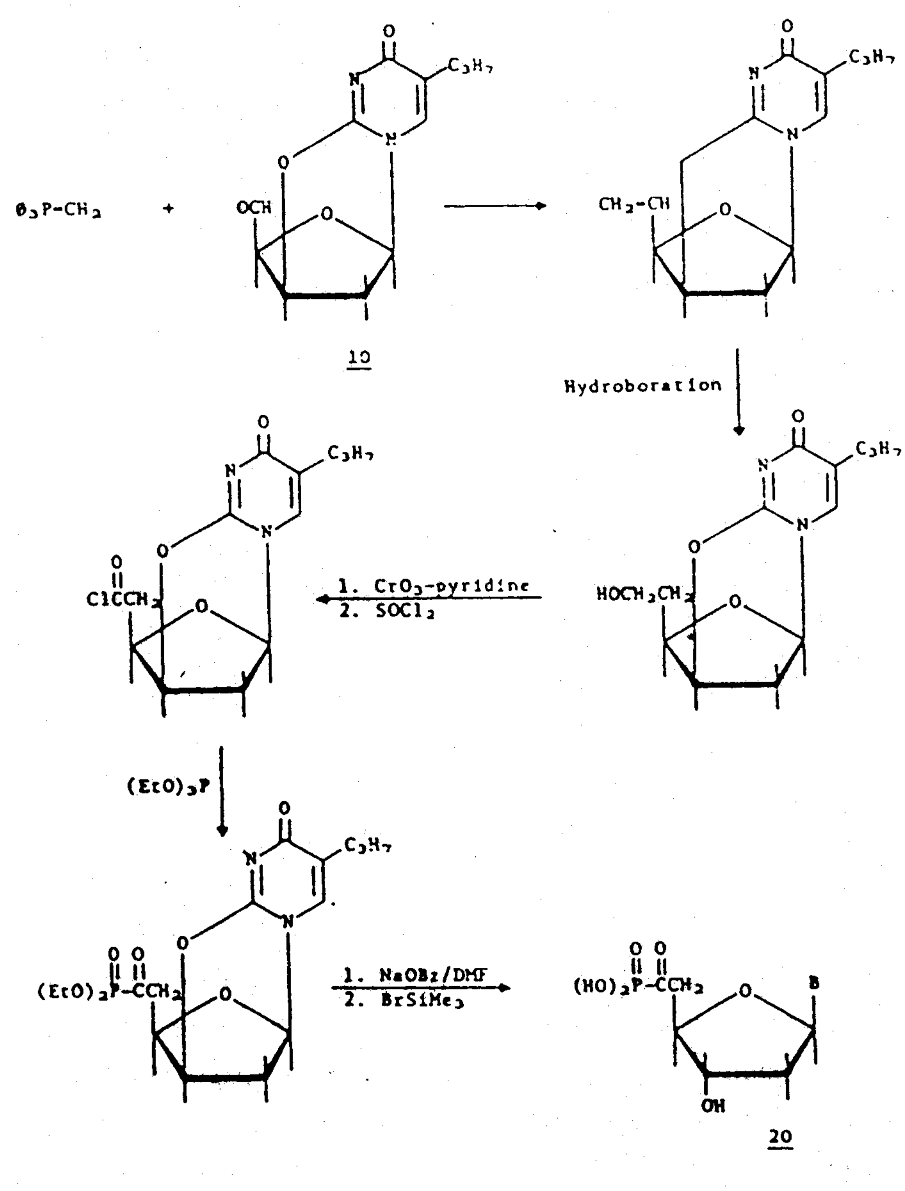


10

20

Scheme VI

Salts

Physiologically acceptable salts of compounds of this invention are prepared by methods known in the art. The salts include ammonium salts and salts of physiologically acceptable metals, particularly $Li^+$, $K^+$, $Na^+$, $Ca^{++}$ and $Mg^{++}$, and are novel compounds and comprise a further aspect of the invention. Metal salts can be prepared by reacting a metal hydroxide with a compound of the invention. Examples of metal salts which can be prepared in this way are salts containing $Li^+$, $Na^+$, and $K^+$. A less soluble metal salt can be precipitated from a solution of a more soluble salt by addition of a suitable metal compound. Acid salts can be prepared by reacting a compound of the invention with an acid such as HCl, Hbr, $H_2SO_4$, or an organic sulphonic acid.

Pharmaceutical Preparations

The compounds of this invention (including the physiologically acceptable salts thereof) have antiviral activity. They present activity against Herpes Simplex viruses and related viruses for example Herpes Simplex virus I, Herpes Simplex virus II, Epstein-Barr virus, varicella Zoster virus, and cytomegalo virus. Thus the compounds can be formulated into pharmaceutical preparations. Such preparations are composed

of one or more of the compounds in association with a pharmaceutically acceptable carrier. The book Remington's Pharmaceutical Sciences, 15th Ed by E.W. Martin (Mark Publ. Co., 1975) discloses typical carriers and methods of preparation, which disclosure is incorporated by reference.

The compounds may be administered topically, orally, parenterally (e.g. intravenously, by intramuscular injection, or by intraperitoneal injection or the like depending upon the nature of the viral infection being treated.

For internal infections the compositions are administered orally or parenterally at dose levels of about 0.1 to 300 mg/kg, preferably 1.0 to 30 mg/kg of mammal body weight and can be used in man in a unit dosage form administered one to four times daily in the amount of 1 to 250 mg per unit dose. For oral administration, fine powders or granules may contain diluting, dispersing and/or surface active agents, and may be presented in water or in a syrup, in capsules or sachets in the dry state or in a nonaqueous solution or suspension, wherein suspending agents may be included; in tablets, wherein binders and lubricants may be included, or in a suspension in water or a syrup. Where desirable or necessary, flavoring, preserving, suspending, thickening or emulsifying agents may be included. Tablets and granules are preferred oral administration forms and these may be coated.

For parenteral administration or for administration as drops, as for eye infections, the compounds may be presented in aqueous solution in a concentration of from about 0.1 to 10%, more preferably about 0.1 to 7%. The solution may contain antioxidants, buffers, etc.

Alternatively, for infections of the eye, or other external tissues, e.g. mouth and skin the compositions are preferably applied to the infecte part of the body of the patient topically as an ointment, cream, aerosol or powder, preferably as an ointment or cream. The compounds may be presented in an ointment, for instance with a water soluble ointment base, or in a cream, for instance with an oil in water cream base in a concentration of from about 0.01 to 10%, preferably 0.1 to 7%, most preferably about 0.5% w/v. Additionally, viral infections of the eye, such as Herpetic Keratitus may be treated by use of a sustained release drug delivery system as is described in the art.

The exact regimen for administration of the compounds and compositions disclosed herein will necessarily be dependent upon the needs of the individual subject being treated, the type of treatment and, of course, the judgement of the attending practitioner.

The invention will be further described by the following nonlimiting examples, to be interpreted with the amendments necessary in view of the claimed subject-matter.

## Example I

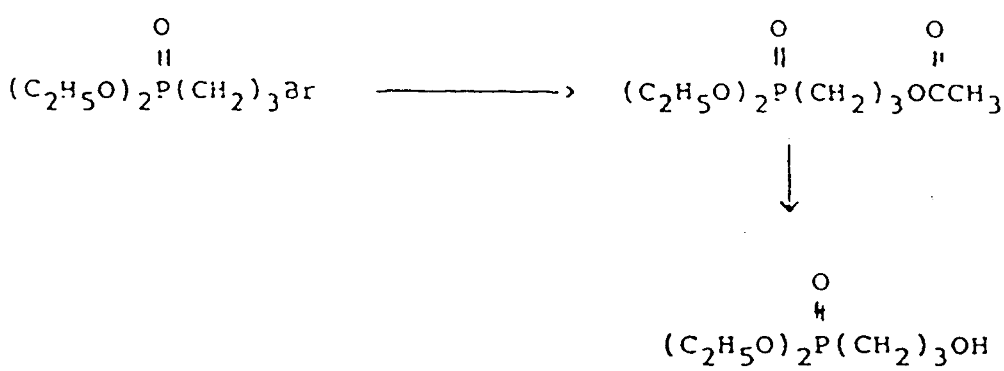

Diethyl-3-hydroxypropylphosphonate

Diethyl-3-bromophosphonate (12.0 g, 46 mmol, prepared by the method of Anatol Eberhard and F.H. Westheimer, JACS 87 253-260 (1965)) was stirred with 12.0 g NaOAc•3$H_2O$ in 125 ml DMF heated in a steam bath. The reaction was evaporated to dryness in vacuo after 2 hours and partitioned between $H_2O$ and EtOAc, extracting the aqueous layer five times. The ethyl acetate extract was washed once with brine, dried with $Na_2SO_4$ filtered, and evaporated to dryness in vacuo to yield 9.8 g light yellow oil (89%). $^1H$ NMR ($CDCl_3$) $\delta$ 1.3 (tr, 6 H), 1.5-2.0 (m, 4 H), 2.03 (s, 3 H), 4.1 (assym. quintet, 6 H) thin layer chromatography on SiGF developed with 2:1 EtOAc:$CH_2Cl_2$ gave $R_f$ 0.30. The isolated diethyl-3-acetoxypropylphosphonate (9.8 g, 41 mmol) in 200 ml abs. EtOH was stirred with 30 ml Dowex 50 ($H^+$) which had

been rinsed three times each with $H_2O$ and EtOH. After 4-1/2 days at room temperature, another 10 ml of similarly prepared resin was added. Six hours later, the reaction was filtered and evaporated in vacuo. The quantitative yield of yellow oil was purified by dry column chromatography on 400 g silica packed in a 2.75 inch flat diameter nylon tube. The column was eluted with 1:9 MeOH:EtOAc and the appropriate fractions were cut and slurried with 1:1 MeOH:EtOAc. Filtration and evaporation in Vacuo afforded 5.33 g (66%) pale yellow oil. $^1H$ NMR ($CDCl_3$ $D_2O$): $\delta$ 1.30 (tr, 6 H)(, 1.60-2.08 (m, 4 H), 3.67 (tr, 2 H), 4.13 (quintet, 4 H); thin layer chromatography on SiGF developed with 1:9 MeOH:EtOAc gave an $R_f$ of 0.57.

9(3-phosphono-1-propyloxymethyl)guanine

To 9.40 mmol silated guanine (James L. Kelley, Mark P. Krochmal, and Howard J. Shaeffer, J Med Chem, 24 1528-1531 (1981)) in 9 ml dry toluene was added 7.60 mmol diethyl-3-chloromethoxypropylphosphonate, prepared from diethyl-3-hydroxypropylphosphonate according to the procedure of Kelley, et al, followed by the addition of 2.2 ml triethylamine. The reaction was refluxed 24 hours and evaporated to dryness in vacuo. The residue was digested with 70 ml EtOH and the voluminous tan solid was isolated by suction filtration. The solid was dissolved in water, made basic with conc. $NH_4OH$, and treated with excess aqueous lead diacetate. The lead salt was isolated by centrifugation and dissolved in 50% acetic acid followed by treatment with $H_2S$ for 20 minutes. The black lead sulfide was removed by suction filtration through Celite. The filtrate was evaporated to dryness in vacuo, triturated in EtOH, and filtered. The residue was further triturated in DMF and filtered to yield 320 mg off-white solid. This solid was dissolved in minimum water, acidified with 1 M HCl. Thereafter it was neutralized with 1 M NaOH and lyophilized. The solid residue was triturated in a mixture of DMF, $H_2O$, and EtOH and filtered to yield 276 mg of 9(3-phosphono-1-propyloxymethyl) quanine as a white solid (8.3%). Anal. ($C_9H_{12}N_5O_5P \cdot 2Na \cdot 5H_2O$) C, H, N; UV $\gamma_{max}$ ($\epsilon$): pH 1, 255 (14, 700); pH 7, 251 (15, 600); pH 11, 257 (12, 800), 267 (12, 800); mass spectrum (TMS derivative) m/e 591 ($M^+$ of $TMS_4$ derivative); $^1H$ NMR ($D_2O$): $\delta$ 1.25-1.90 (m, 4 H), 3.42 (tr, 2 H), 5.50 (s, 2 H), 7.92 $\delta$ (s, 1 H). Thin layer chromatography on SiGF developed with 7:3 $CH_3CN$:0.1 N $NH_4Cl$ gave $R_f$ 0.20.

## Example II

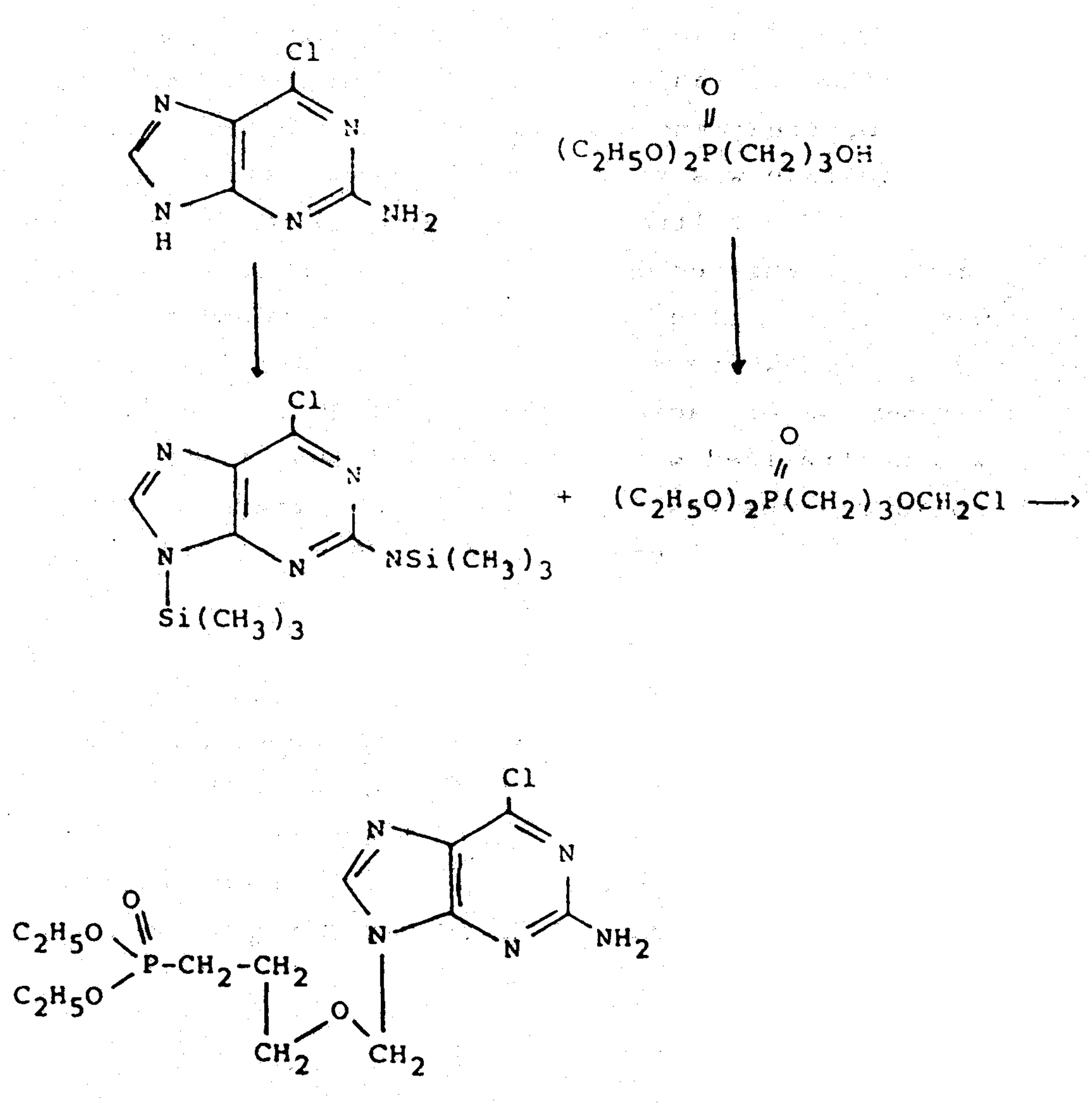

6-Chloro-9(3-diethylphosphono-1-propyloxymethyl)guanine

To 0.5 g (2.95 mmol) 2-amino-6-chloropurine, silated and treated with $Hg(CN)_2$ according to the procedure of Robins and Hatfield (Morris J. Robins and Peter W. Hatfield, Can J Chem, 60 547-553 (1982)) in 40 ml benzene was added a solution of 2.68 mmol diethyl-3-chloromethoxypropylphosphonate prepared from (0.525 g) diethyl-3-hydroxypropylphosphonate according to the procedure of Kelley, et al, (James L. Kelley, Mark P. Krochmal and Howard J. Shaeffer, J Med Chem, 24 1528-1531 (1981)). The reaction was refluxed for 2 hours, cooled and 400 ml $CHCl_3$ was added. The organic phase was washed successively with 80 ml each of aqueous saturated $NaHCO_3$ and 1 M aqueous KI. The organic solution was dried over $Na_2SO_4$, filtered and evaporated to 790 mg of yellow gum. A portion of this crude material was used to conduct hydrolysis experiments. The remaining material was chromatographed on a silica column. A solution of 574 mg of the crude reaction product was placed on 20 g silica packed in a column using 5:3 EtOAc:nPrOH. Elution with the same mixed solvent afforded sixteen fractions of 10-20 ml each. Fractions 7-12 were combined to yield 258 mg of a colorless oil which spontaneously crystallized. Trituration in $CH_2Cl_2 \cdot Et_2O$ afforded two crops of white solid (207 mg), mp 109-110° (28%). A yield of 46% was obtained from a reaction performed on 45.2 mmol of the starting purine. Anal ($C_{13}H_{21}ClN_5O_4P$) $C_1H_1N$; UV $\gamma_{max}$ ($\epsilon$): pH 1, 246 ($\epsilon$ 6600), 310 (7200); pH 7, 247 (6800), 308 (7400); pH 11 247 (6600), 308 (7100); mass

spectrum: m/e 377 ($M^+$); $^1$H NMR ($CDCl_3$): δ 1.3 (tr, 6 H), 1.52-2.18 (m, 4 H), 3.58 (tr, 2 H), 4.09 (qu, 4 H), 5.48 (s, with broad base, 4 H), 7.89 (s, 1 H). Thin layer chromatography on SiGF developed with 5:3 EtOAc:nPrOH gave $R_f$ 0.40.

9(3-ethylphosphono-1-propyloxymethyl)guanine

6-Chloro-9(3-diethylphosphono-1-propyloxymethyl)guanine (75 mg; 0.2 mmol) was combined with 5 ml 1 N aqueous NaOH and refluxed 1 hour. The cooled reaction was neutralized with Dowex 50X8 (pyridinium form) and filtered, rinsing liberally with water. The solution was partially evaporated to remove pyridine and was then lyophilized. The orange-colored residue (74 mg) was redissolved in $H_2O$ and centrifuged to remove insoluble material. The decanted solution (2 ml) was chromatographed on a 0.9 x 46 cm column of Whatman DE-52 Cellulose, $HCO_3$ form, using a linear gradient of one liter each $H_2O$ and 0.2 M $NH_4HCO_3$ after an initial $H_2O$ elution. Fractions (7 ml each) 43-47 yielded 25 mg (36%) of fluffy white solid after three lyophilizations. Electron impact mass spectrum (TMS derivative) showed m/1 547 ($M^+$ of TMS derivative); chemical ionization mass spectrum (TMS derivative) showed m/1 548 ($M^+$ + H of $TMS_3$ (derivative). $^1$H NMR ($D_2O$) showed δ 1.19 (tr, 3 H), 1.4-1.9 (m, 4H), 3.59 (tr. 2 H), 3.90 (quintet, 2 H), 5.47 (s, 2 H), 8.2 (br•s, 1 H). Thin layer chromatography behavior on SiGF: Rf 0.40 when developed with 7:3 $CH_3CH$. 0.1 N aqueous $NH_4Cl$. The material had a formula of ($C_{11}H_{18}N_5O_5P \cdot H_2O$) Calc: C-37.82% H-5.77%, N-20.0. Found: C-38.27%, H-5.84%, N-19.65%. A UV spectum was run on the material and showed UV $\gamma_{max}$ (ε: pH 1, 256 (10, 400), 278 shoulder; pH 7, 252 (11, 300) 271 shoulder; pH 11, 256-258 (9, 600) 267 shoulder. The product was relyophilized.

Example III

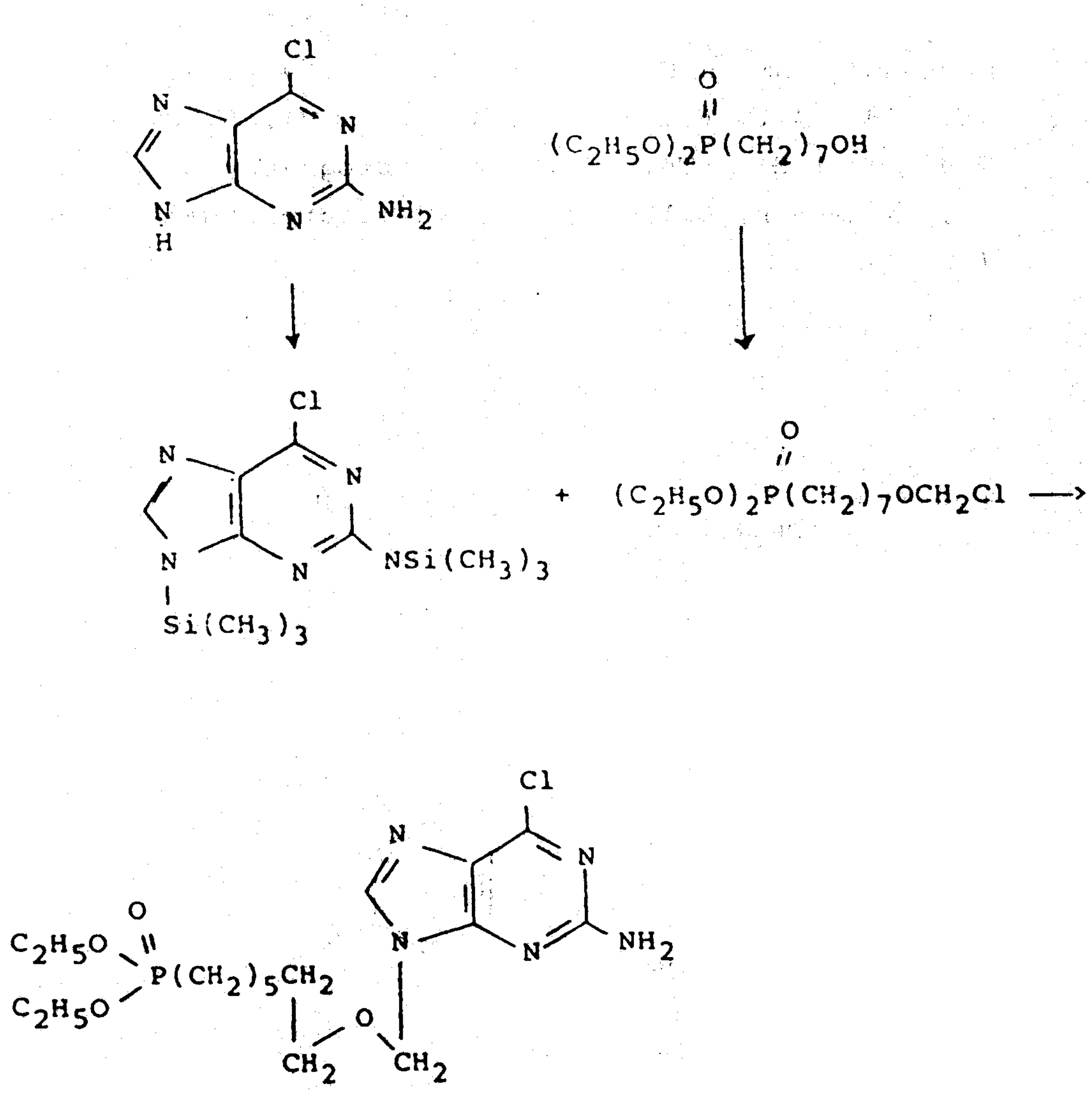

6-Chloro-9(7-diethylphosphono-1-heptyl-methyl)guanine

Diethyl 7-chloromethoxyheptylphosphonate was prepared from 1,7-dibromoheptane and triethylphosphite by the procedures used to prepare diethyl 3-chloromethoxypropylphosphonate (Example I). It was reacted with silated 2-amino-6-chloropurine, and mercuric cyanide as described for the preparation of 6-chloro-9(3-diethylphosphono-1-propyloxymethyl)guanine (Example II) to give 32% of product as a colorless gum. UV $\gamma_{max}$ pH 1: 246 nm ($\epsilon$ 6220), 310 nm ($\epsilon$ 6380); $\gamma_{max}$ pH 7, 247 nm ($\epsilon$ 5910), 310 nm ($\epsilon$ 6410); $\gamma_{max}$ pH 11, 246 nm ($\epsilon$ 5950), 309 nm ($\epsilon$ 6380); mass spectrum 'H NMR ($CDCl_3$) ∫ 1.1-1.9 (m, 18 H), 3.48 (t, 2 H), 4.10 (q, 4 H), 5.47 (s, 2 H), 5.88 (s, 2 H), 7.93 (s, 1 H). Thin layer chromatography on silica gel GF gave $R_f$ 0.15 using ethylacetate:ethanol (100:1).

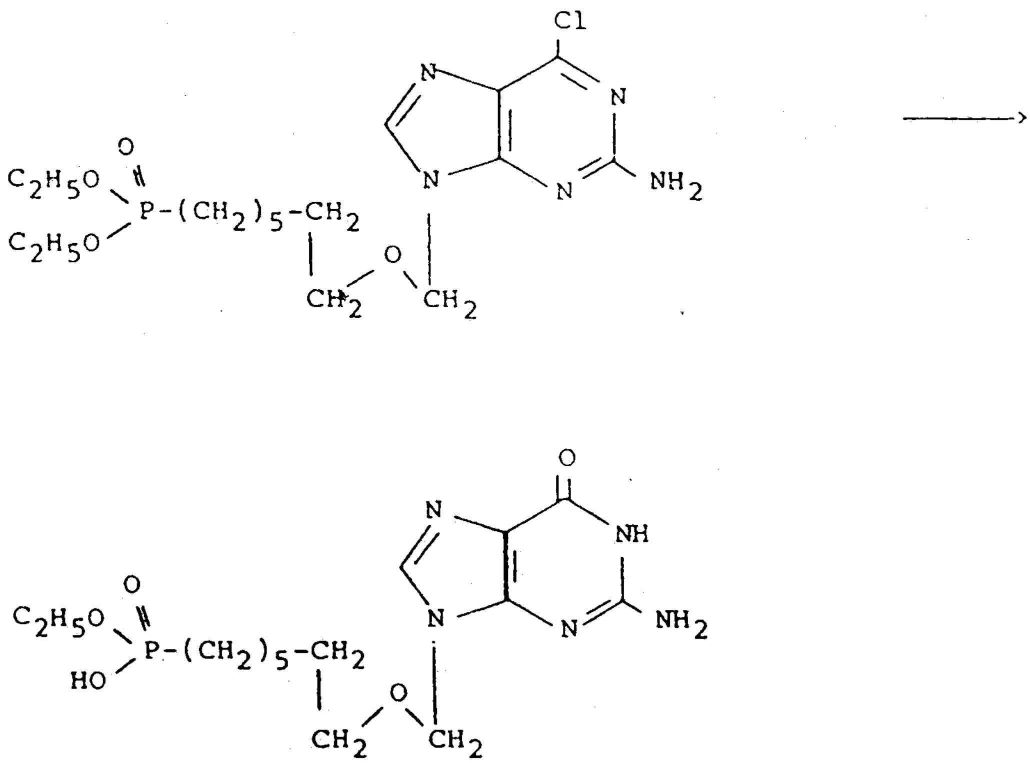

9-(7-ethylphosphono-1-heptyloxymethyl)guanine:

6-chloro-9(7-diethylphosphono-1-heptyloxy-methyl)guanine was hydrolyzed by refluxing 1 N aqueous sodium hydroxide for 4 hours and isolated in 30% yield as described for the preparation of 9-(3-ethylphosphono-1-propyloxymethyl)guanine (Example II). It has $R_f$ 0.5 on silica gel GF using acetonitrile (7:3) 0.1 N aqueous ammonium chloride. Proton NMR ($D_2$ O) 1.1-1.5 (m, 15 H), 3.5 (t, 2 H), 3.90 (q, 2 H), 5.45 (s, 2 H) UV.

Biological Testing

The compounds of Example I and II were evaluated in vitro as antiviral agents against herpes virus.

The virus strain employed was Strain McCrae of type 1 herpes (thymidine kinase positive virus) prepared and titered in MA-104 cells and frozen at -90 °C until use.

Continuous passaged monkey kidney (MA-104) cells were used, with growth medium consisting of Minimum Essential Medium (MEM) supplemented with 0.1% $NaHCO_3$ and 9% fetal calf serum. Test medium consisted of MEM supplemented with 2% fetal bovine serum, 0.18% $NaHCO_3$ and 50 $\mu$l gentamicin.

The last compounds were added to test medium at a concentration of 2000 $\mu$g/ml for use as a positive control.

Antiviral Test Method

To a 96 well microtiter plate containing an established 24 hour monolayer of cells from which the medium has been decanted was added 0.1 ml of varying (one-half $\log_{10}$) concentrations of test compound, which incubated on the cell 15 minutes, after which 0.1 ml of virus in a concentration of 320 cell culture 50% infectious doses (CCID50)/0.1 ml was added. The plate was covered with plastic wrap and incubated at 37 °C. Included with the test were toxicity controls (each concentration of compound + test medium in place of compound) and cell controls (test medium in place of compound and virus). The cells were examined microscopically after 72 hours for evidence of cytotoxicity and for viral cytopathic effect (CPE). Vidarabine was run on the same plate in parallel.

Antiviral activity was determined by observation of inhibition of viral CPE. This activity was expressed

by minimum inhibitory concentration (MIC), Defined as that dose range of compound causing 50% CPE inhibition. A Virus Rating (VR) was also determined, which is a numerical expression of antiviral activity, weighted to take into account any cytotoxicity observed. Generally, a VR of 0.1 - 0.4 is usually indicative of slight antiviral effect, 0.5 - 0.9 indicates moderate antiviral effedt, and ≥1.0 implies strong antiviral activity.

The results of these are summarized in tables A and B. The test compound has a strong activity against the thymidine kinase positive type I herpes virus. The activity was considered equivalent to that of vidarabine.

TABLE A

Effect of compound of Example I and Vidarabine on Thymidine Kinase-Positive Type 1 Herpes Virus Infections in MA-104 Cells

| Compound of I: Conc. (μg/ml | Compound of I: CPE[a] Inhib. (%) | Vidarabine: Conc. (μ/ml) | Vidarabine: CPE[a] Inhib. (%) |
|---|---|---|---|
| 1000 | 100 | 1000 | 100 |
| 320 | 94 | 320 | 100 |
| 100 | 79 | 100 | 87 |
| 32 | 62 | 32 | 87 |
| 10 | 49 | 10 | 69 |
| 3.2 | 28 | 3.2 | 28 |
| 1.0 | 31 | 1.0 | 56 |
| 0 | | 0 | |
| VR[b] | 1.4 | 1.3 | |
| MIC[c] | 10 | 10 | |

[a]Cytopathic effect, % cell alteration or destruction.

[b]Virus rating, a numerical expression of antiviral activity (Sidwell et al, Appl Microbiol, 22:797, (1971), 0.1 - 0.4 = slight activity, 0.5 - 0.9 = moderate activity, ≥ 1.0 = strong activity.

[c]Minimum inhibitory concentration - that dosage range wherein a 50% CPE inhibition is seen.

TABLE B

Effect of compound of Example II and Vidarabine on Thymidine Kinase Positive Type 1 Herpes Virus in MA-104 Cells (Two Tests)

| Compound of II | | | Vidarabine (Control) | | |
|---|---|---|---|---|---|
| Conc. (μg/ml | Test1 CPE[a] Inhib. (%) | Test2 CPE[a] Inhib. % | Conc. (μ/ml) | Test1 CPE[a] Inhib. (%) | Test2 CPE[a] Inhib. (%) |
| 1000 | 100 | 76 | 1000 | 100 | 100 |
| 320 | 82 | 67 | 320 | 100 | 100 |
| 100 | 47 | 57 | 100 | 96 | 85 |
| 32 | 6 | 57 | 32 | 96 | 57 |
| 10 | 38 | 48 | 10 | 60 | 39 |
| 3.2 | 96 | 52 | 3.2 | 2 | 0 |
| 1.2 | 69 | 48 | 1.0 | 0 | 0 |
| 0 | | | 0 | | |
| VR[b] | >2.0 | >1.4 | VR[b] | 0.8 | 07 |
| MIC[c] | <1.0 | <1.0 | MIC[c] | 10 | 10 |
| MTD[d] | 320 | >1000 | | 10 | 10 |

[a]Cytopathic effect, % cell alteration or destruction.

[b]Virus rating, a numerical expression of antiviral activity (Sidwell et al, Appl Microbiol 22:797, (1971), 0.1 - 0.4 = slight activity, 0.5 - 0.9 = moderate activity, ≥ 1.0 = strong activity.

[c]Minimum inhibitory concentration - that dosage range wherein a 50% CPE inhibition is seen, μg/ml.

[d]Maximum tolerated dose, μg/ml.

The compound of Example II was further evaluated by the above-described in vitro test method against cytomegalo virus. The compound was strongly active with a VR of 2.1-2.3.

The compound of Example II was tested in vivo in guinea pigs as an agent against thymidine kinase positive herpes virus. The animals were innoculated with the virus. Eighteen hours later the material of Example II (0.4% solution in water or 1-2% solution), a 5% solution of acyclovir or a 1.4% solution of poly-(vinylalcohol) was administered and five days later blister diameters at the point of innoculation were measured. Satellite lesions were measured as well.

The results of these tests are given in Table C and show that the compound has superior activity against $TK^+$ virus.

TABLE C

Effect of Compound of Example II
in vivo against Herpes Virus

| Virus | Placebo, 1.4% poly(vinylalcohol) | Acyclovir, 5% | Cx of II 1-2%* | Cx of II 0.4% |
|---|---|---|---|---|
| $TK^{+}$ | 1.7** | 1.0 | 0.9 | 2.1 |
| Satellite lesions | 9 | 4 | 6 | 11 |

*saturated solution

**average number of lesions

Formulations

The following formulations based on the compounds of the invention and their preparation are representative.

A formulation suitable for injections intramuscularly or intraperitoneally is prepared by combining the first four of the following materials

| | |
|---|---|
| Compound of the Invention | 1 gram |
| Poly(ethylene glycol) | 50 grams |
| Propylene glycol | 50 grams |
| Tween 80 suspension agent | 1.5 grams |
| Injectable Saline | 200 ml |

and then adding the last material. The material forms a clear solution which is filtered and sealed in sterile containers.

A simple intravenous injection formulation is formed by dissolving 1 gram of an active compound in 250 ml of injectable saline which after filtering is packaged in sterile bottles.

A cream for topical administration is formulated by stirring 10 g of active compound of the invention with 20 g of mineral oil, 40 g of petroleum jelly, 0.3 g of mixed methyl/propyl paraben and 5 g of nonionic surfactant at 50 ° C. Then 150 ml of water are stirred into the mixture at 50 ° C at high speed to form a cream and the mixture is cooled and packaged in capped tubes.

An oral dosage form is prepared from 10 g of compound of the invention, 100 g of lactose, and 1 g of starch which are mixed with 0.1 g of magnesium stearate in methanol to granulate. The methanol is removed by gentle heating with stirring. A portion of this material is retained as a granular powder for oral use while the remainder is hand formed into 250 mg tablets in a manual tableting machine.

The foregoing examples and formulations have been presented to illustrate the present invention and are not to be construed as limitations on the invention's scope which is instead defined by the following claims.

**Claims**

**1.** A compound having the structure

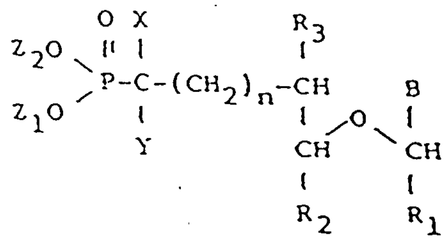

wherein $Z_1$ and $Z_2$ are the same or different and selected from the group made up of hydrogen, the one to six carbon alkyls, phenyl and benzyl, X is H, OH, or together with Y = O, Y is H or together with X = O, n is an integer, 0, 2 or 4, $R_1$ and $R_2$ together complete a beta-pentofuranose sugar or $R_1$ is H and $R_2$ is H or $-CH_2OH$, $R_3$ is H or OH and B is a pyrimidine base selected from 5-iodouracil, 5-trifluorothymine, 5-iodocytosine, E-5-(2 bromo-vinyl) uracil, 5-propyluracil and 5-ethyluracil.

**2.** The compound of claim 1 wherein the beta-pentofuranose is a beta-ribofuranose.

**3.** The compound of claim 1 wherein the beta-pentofuranose is a beta-arabinofuranose.

**4.** The compound of claim 1 wherein $R_1$ is hydrogen and $R_2$ is hydrogen.

**5.** The compound of claim 1 wherein $R_1$ is hydrogen and $R_2$ is hydroxymethyl.

**6.** The compound of any one of claims 1 to 5 wherein $Z_1$ and $Z_2$ are each selected from the group made up of hydrogen and one to four carbon alkyls.

**7.** The compound of any one of claims 1 to 6 wherein X and Y are each hydrogens.

**8.** The compound of any one of claims 1 to 6 wherein X and Y are = O.

**9.** The compound of any one of claims 1 to 6 wherein X is hydroxyl and Y is hydrogen.

**10.** The compound of any one of claims 1 to 9 wherein n is 0.

**11.** The compound of any one of claims 1 to 9 wherein n is 2.

**12.** The compound of any one of claims 1 to 9 wherein n is 4.

**13.** A metal salt of a compound of claim 1.

**14.** A pharmaceutical preparation comprising a compound of claim 1 in a pharmaceutically acceptable carrier.

**15.** A compound according to any one of claims 1 to 11 for use in treating herpes in a mammal.

## Patentansprüche

**1.** Verbindung der Struktur

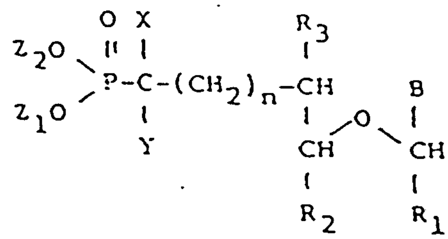

worin $Z_1$ und $Z_2$ gleich oder verschieden und aus der aus Wasserstoff, Alkylgruppen mit 1 bis 6 Kohlenstoffatomen, Phenyl und Benzyl bestehenden Gruppe ausgewählt sind, X H oder OH ist oder zusammen mit Y = O, Y H ist oder zusammen mit X = O, n eine ganze Zahl, 0, 2 oder 4 bedeutet, $R_1$ und $R_2$ gemeinsam einen β-Pentofuranosezucker vervollständigen oder $R_1$ H und $R_2$ H oder $-CH_2OH$ ist, $R_3$ H oder OH ist und B eine Pyrimidinbase, ausgewählt aus 5-Joduracil, 5-Trifluorthymin, 5-Jodcytosin, E-5-(2-Bromvinyl)-uracil, 5-Propyluracil und 5 Ethyluracil, ist.

**2.** Verbindung nach Anspruch 1, worin die β-Pentofuranose eine β-Ribofuranose ist.

**3.** Verbindung nach Anspruch 1, worin die β-Pentofuranose eine β-Arabinofuranose ist.

**4.** Verbindung nach Anspruch 1 , worin $R_1$ Wasserstoff und $R_2$ Wasserstoff ist.

**5.** Verbindung nach Anspruch 1, worin $R_1$ Wasserstoff und $R_2$ Hydroxymethyl ist.

**6.** Verbindung nach irgendeinem der Ansprüche 1 bis 5, worin $Z_1$ und $Z_2$ jeweils aus der aus Wasserstoff und Alkylgruppen mit 1 bis 4 Kohlenstoffatomen bestehenden Gruppe ausgewählt sind.

**7.** Verbindung nach irgendeinem der Ansprüche 1 bis 6, worin X und Y jeweils Wasserstoffatome sind.

**8.** Verbindung nach irgendeinem der Ansprüche 1 bis 6, worin X und Y = O sind.

**9.** Verbindung nach irgendeinem der Ansprüche 1 bis 6, worin X Hydroxyl und Y Wasserstoff ist.

**10.** Verbindung nach irgendeinem der Ansprüche 1 bis 9, worin n 0 ist.

**11.** Verbindung nach irgendeinem der Ansprüche 1 bis 9, worin n 2 ist.

**12.** Verbindung nach irgendeinem der Ansprüche 1 bis 9, worin n 4 ist.

**13.** Metallsalz einer Verbindung von Anspruch 1.

**14.** Pharmazeutisches Präparat, umfassend eine Verbindung nach Anspruch 1 in einem pharmazeutisch annehmbaren Träger.

**15.** Verbindung nach irgendeinem der Ansprüche 1 bis 11 zur Verwendung bei der Behandlung von Herpes bei Mensch und Säugetieren.

## Revendications

**1.** Composé répondant à la formule

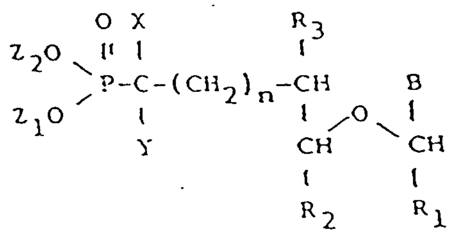

dans laquelle $Z_1$ et $Z_2$ sont identiques ou différents et sont choisis dans le groupe comprenant l'hydrogène, les restes alkyle ayant un à six atomes de carbone, phényle et benzyle, X représente H, un groupe OH ou forme conjointement avec Y un atome d'oxygène, Y représente H ou forme conjointement avec X un atome d'oxygène, n est un nombre entier égal à 0, 2 ou 4, $R_1$ et $R_2$ complètent conjointement un sucre du type bêta-pentofurannose ou bien $R_1$ représente H et $R_2$ représente H ou le groupe $-CH_2OH$, $R_3$ représente H ou le groupe OH et B est une base pyrimidique choisie entre le 5-iodouracile, la 5-trifluorothymine, la 5-iodocytosine, le E-5-(2-bromovinyl)-uracile, le 5-propyluracile et le 5-éthyluracile.

**2.** Composé suivant la revendication 1, dans lequel le bêta-pentofurannose est un bêta-ribofurannose.

**3.** Composé suivant la revendication 1, dans lequel le bêta-pentofurannose est un bêta-arabinofurannose.

**4.** Composé suivant la revendication 1, dans lequel $R_1$ est l'hydrogène et $R_2$ est l'hydrogène.

**5.** Composé suivant la revendication 1, dans lequel $R_1$ est l'hydrogène et $R_2$ est le groupe hydroxyméthyle.

**6.** Composé suivant l'une quelconque des revendications 1 à 5, dans lequel $Z_1$ et $Z_2$ sont tous deux choisis dans le groupe formé de l'hydrogène et des restes alkyle ayant un à quatre atomes de carbone.

**7.** Composé suivant l'une quelconque des revendications 1 à 6, dans lequel X et Y sont tous deux des atomes d'hydrogène.

**8.** Composé suivant l'une quelconque des revendications 1 à 6, dans lequel X et Y représentent =O.

**9.** Composé suivant l'une quelconque des revendications 1 à 6, dans lequel X est un groupe hydroxyle et Y est l'hydrogène.

**10.** Composé suivant l'une quelconque des revendications 1 à 9, dans lequel n est égal à 0.

**11.** Composé suivant l'une quelconque des revendications 1 à 9, dans lequel n est égal à 2.

**12.** Composé suivant l'une quelconque des revendications 1 à 9, dans lequel n est égal à 4.

**13.** Sel métallique d'un composé suivant la revendication 1.

**14.** Préparation pharmaceutique, comprenant un composé de formule 1 dans un support pharmaceutiquement acceptable.

**15.** Composé suivant l'une quelconque des revendications 1 à 11, destiné à être utilisé dans le traitement de l'herpès chez un mammifère.